# EUROPEAN PATENT APPLICATION

(11) **EP 3 025 642 A1**
(43) Date of publication of application: **01.06.2016**
(21) Application number: 15196832.8
(22) Date of filing: 27.11.2015
(51) Int. Cl.: A61B 5/00

(54) **SUBJECT INFORMATION ACQUIRING APPARATUS**

(30) Priority: 28.11.2014 JP 2014242443
(71) Applicant: CANON KABUSHIKI KAISHA, Ohta-ku Tokyo 146-8501 (JP)
(72) Inventor: AZUMA, Masataka, Kyoto-Shi, Kyoto 606-8501 (JP); SHIINA, Tsuyoshi, Kyoto-Shi, Kyoto 606-8501 (JP); NAMITA, Takeshi, Kyoto-Shi, Kyoto 606-8501 (JP); YAMAKAWA, Makoto, Kyoto-Shi, Kyoto 606-8501 (JP); KONDO, Kengo, Kyoto-Shi, Kyoto 606-8501 (JP); NAGAE, Kenichi, Ohta-ku Tokyo 146-8501 (JP)
(74) Representative: Houle, Timothy James

(57) **Abstract**

A subject information acquiring apparatus includes a light source (005) configured to radiate optical pulses coded by a predetermined number of pulse streams to a subject (000), a probe (003) having a converting element configured to receive photoacoustic waves occurring from the subject (000), and configured to convert the received photoacoustic waves to electrical signals, and an information acquiring unit configured to decode a predetermined number of signals of the electrical signals output from the converting element and acquire characteristic information on the subject (000) based on the decoded electrical signals, wherein the relative positions of the converting element included in the probe (003) and the subject (000) are changed before the radiation of the predetermined number of optical pulse streams completes, and the electrical signals to be decoded are partially redundant before and after the change of the relative positions of the converting element and the subject (000).

## Description

### Field of the Invention

The present invention relates to a subject information acquiring apparatus which irradiates light to a subject, detects acoustic waves occurring from inside of the subject, and images the inside of the subject are obtained.

### Description of the Related Art

Optical imaging apparatuses which may irradiate light from a light source such as a laser to a subject such as a living body, and image information regarding the inside of the subject based on the incident light, have been actively studied in medical fields. Photoacoustic Imaging (PAI) is one of such optical imaging technologies. Photoacoustic imaging irradiates pulsed light occurring from a light source to a subject, and detects acoustic waves (typically ultrasonic waves) occurring from a tissue of the subject that absorbs energy of the pulsed light propagated and diffused inside the subject. Based on detected signals therefrom, internal information of the subject is imaged.

H. Zhang K. Kondo, M. Yamakawa, And T. Shiina: Proc. SPIE 8581 PhotonsPlus Ultrasound: Imaging and Sensing 2013, (2013) 85812Y discloses a technology which irradiates light at a time point based on Periodic and unipolar M-sequences (PUMs) and decodes received photoacoustic signals for improving the signal-to-noise ratio of the received signals. Japanese Patent No. 5210087 discloses an apparatus which irradiates light by using an amplifying unit configured to amplify light from a semiconductor laser pulsed light source, performs correlation processing on signals for the light irradiation and received photoacoustic signals, and acquires a tomographic image based on the signals resulting from the correlation processing.

However, such a photoacoustic image reconstruction includes an important problem of inhibition of artifacts occurring during image reconstruction processing, in addition to the problems of improvement of the signal-to-noise ratio of received photoacoustic signals (hereinafter, simply called received signals).

The artifacts occurring during the reconstruction processing also includes an artifact caused by insufficient directions and positions for receiving photoacoustic waves. In order to inhibit such artifacts, an increase of directions and positions of receiving photoacoustic waves may be necessary. However, the increase of directions and positions may increase the size of the whole system due to the increased imaging time and the increased number of receiving elements therefor, improvement of which has also been demanded.

The present invention provides a subject information acquiring apparatus capable of acquiring information in a short period of time at increased numbers of directions and positions of receiving, and reducing artifacts without increasing the size of the whole system in which the apparatus is included.

### SUMMARY OF THE INVENTION

According to an aspect of the present invention a subject information acquiring apparatus as specified in claims 1 to 11 is provided.

Further features of the present invention will become apparent from the following description of embodiments with reference to the attached drawings. Each of the embodiments of the present invention described below can be implemented solely or as a combination of a plurality of the embodiments or features thereof where necessary or where the combination of elements or features from individual embodiments in a single embodiment is beneficial.

### BRIEF DESCRIPTION OF THE DRAWINGS

Fig. 1 illustrates a system overview of a subject information acquiring apparatus according to an embodiment of the present invention.
Fig. 2 schematically illustrates time points for irradiating optical pulses and light irradiation operations.
Figs. 3A and 3B illustrate a comparison between an image acquired according to an embodiment of the present invention and a comparison image.
Figs. 4A and 4B illustrate examples of an embodiment of the present invention.
Fig. 5 illustrates a system overview of a subject information acquiring apparatus according to another embodiment of the present invention.

### DESCRIPTION OF THE EMBODIMENTS

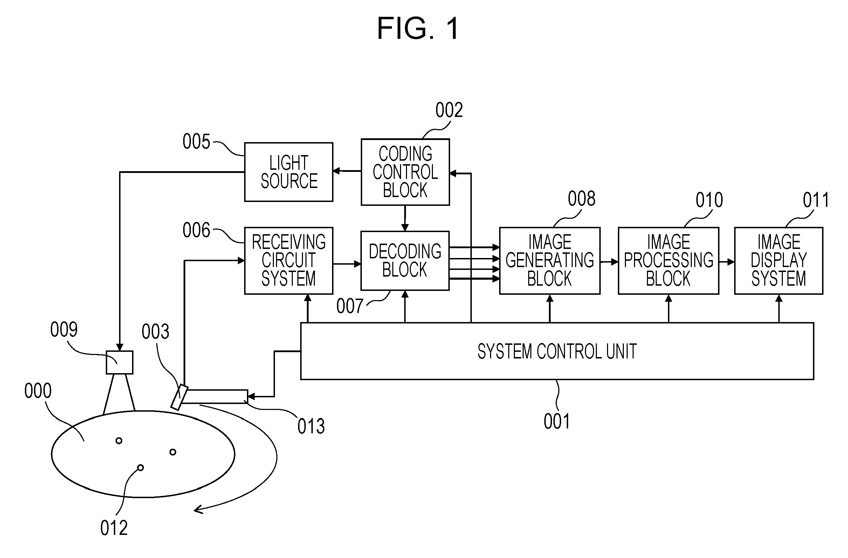

Fig. 1 illustrates a system overview of a subject information acquiring apparatus according to an embodiment of the present invention. With reference to Fig. 1, a signal flow in the apparatus will be described, and processes to be performed in the flow will then be described in detail.

In accordance with a control signal from a system control unit 001, a coding control block 002 generates a predetermined number of code string signals, and inputs the code string signals to a light source 005. The light source 005 generates optical pulses in accordance with the input code string signals. In other words, the light source 005 generates optical pulses coded by the predetermined number of pulse streams. The optical pulses are input to a light irradiating unit 009, and the light irradiating unit 009 irradiates the optical pulses to a target position of a subject 000.

The optical pulses are diffused and propagated inside the subject 000 and are absorbed by an absorbent 012 inside the subject. The absorbent 012 generates photoacoustic waves depending on a physical property value (such as an absorption coefficient and a Gruneisen coefficient) and the light intensity thereof. The photoacoustic waves generated by the absorbent 012 are converted to electrical signals (such as a voltage signal) by converting elements (such as a piezoelectric element and a capacitative converting element) included in a probe 003, and the electric signals are input to a receiving circuit (receiving circuit system) 006 as received signals. A transfer mechanism 013, being a scanning unit, supports the probe 003 and changes the relative position of the probe 003 about the subject 000 while optical pulses are being irradiated and photoacoustic waves are being received. The receiving circuit system 006 amplifies and converts voltage signals being the received signals to digital signals. The digital signals are output from the receiving circuit system 006 to a decoding block 007. The decoding block 007 receives information regarding the code string signals output from the coding control block 002. The information regarding the input digital signals and the code string signals is used to execute a plurality of decoding processes, and a plurality of decoded signals are calculated and output from the decoding block 007 and input to an image generating block 008.

The image generating block 008 performs an image reconstruction based on the plurality of decoded signals, and information regarding a receiving position input from the system control unit 001 to acquire a signal (photoacoustic signal) corresponding to the position of interest within the subject. The image generating block 008 calculates a reconstruction image based on the photoacoustic signals and outputs the reconstruction image to an image processing block 010. According to this embodiment, the decoding block 007 and the image generating block 008 configure an information acquiring unit. The image processing block 010 may adjust a dynamic range and perform an image process using a low pass filter, or an image filter for edge emphasis processing, as required, and outputs the generated image brightness data to an image displaying unit (image display system) 011. The image display system 011 displays the input image brightness data in accordance with an instruction from the system control unit 001.

The fundamental configuration and signal flow of the subject information acquiring apparatus according to the present invention have been described.

Next, operations to be performed by the coding control block 002 will be described.

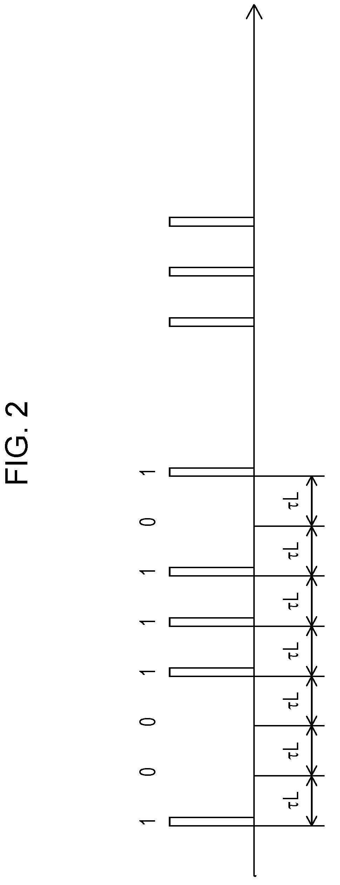

The coding control block 002 generates a code string for driving a light source. The code string to be used has a negative component replaced by 0 in an M-sequence code string generated by using a linear feedback shift register as disclosed in H. Zhang K. Kondo, M. Yamakawa, And T. Shiina: Proc. SPIE 8581 PhotonsPlus Ultrasound: Imaging and Sensing 2013, (2013) 85812Y. For example, when the M-sequence code string is [1 -1 -1 1 1 1 -1], the coding control block 002 generates a code string (bit string) [1 0 0 1 1 1 0]. The light source outputs optical pulses in accordance with the code string. Assuming that the independently predetermined time period between pulses is τL, the light source radiates an optical pulse based on "1" at the first bit but does not radiate an optical pulse based on "0" at the second bit after the time period τL. Then, after a time period of 3 x τL, the light source radiates an optical pulse based on "1" at the fourth bit. After a time period of 6 x τL, the light source does not radiate an optical pulse based on "0" at the seventh bit and, after a time period of 7 x τL, radiates an optical pulse based on the first bit. In this system as described above, the light source 005 is controlled by repetitively using a predetermined number of (7 in this case) code strings so as to radiate pulsed light to the subject 000. Fig. 2 schematically illustrates irradiation timing and light irradiation operations. The coded irradiation of optical pulses is performed by changing the relative positions of the converting elements included in the probe and the subject before the irradiation of a predetermined number of (7 in the example above) optical pulse streams finishes. The relative positions of the converting element and the subject may be changed every 1-bit irradiation, but the present invention is not limited thereto.

Assume that aₖᵢ (k = 1, 2, ..., n, i = 1, 2, ..., m) is a code string to be used here. In this case, n is the number of bits in the code string, and m indicates the number of times that the code string has repeatedly and serially been used. For example, after τL of aₙ₁, code a₁₂ is used to control the light source 005.

Next, operations to be performed by the decoding block 007 will be described.

The decoding block 007 performs decoding processing on a digital signal that decoding block 007 has received.

For the code string [1 0 0 1 1 1 0] above, [1 -1 -1 1 1 1 -1] is used for decoding. The decoding is performed by using signals (electrical signals) corresponding to the number of (7 here) decode strings, which are equal to the number of code strings (7, here) for coding. In other words, when optical pulses coded with a predetermined number of pulse streams are irradiated to a subject, signals based on acoustic waves generated from the irradiated light indicate electrical signals corresponding to an equal number of (7 here) decode strings, which is to be used for decoding. Here, assume that the time period for receiving photoacoustic waves from a region (the position of interest within the subject) for acquiring a photoacoustic signal after pulsed light is irradiated is TR (typically a time period required for propagating ultrasonic waves by a distance from the position farthest from the probe in the region for acquiring the photoacoustic signals to the probe). After the irradiation of light corresponding to a predetermined number (n here, n = 7) of code strings completes (after (n - 1) x time period τL), signals which have been continuously received by the time period TR are decoded by using the decode strings. More specifically, the received digital signals are decoded by calculating a correlation with the decode strings corresponding to the repetition period (τL) of the pulsed light.

The decoding block further performs decoding processing on a digital signal delayed by 1 bit, that is, τL (a signal acquired by performing digital conversion on a received signal corresponding to acoustic waves received in a period of (n-1) x τL + TR from the second light irradiation) by using a code string [-1 -1 1 1 1 -1 1]. A code string (a21, a31, ... an1, a12) is used here for light irradiation. In other words, in decoding using a predetermined number of electrical signals performed by the decoding block 007 included in the information acquiring unit, because the relative positions of the converting elements included in the probe and the subject are changed before the irradiation of a predetermined number of optical pulse streams finishes, the electrical signals used for the decoding are partially redundant before and after the change of the relative positions of the converting element and the subject. The predetermined number of electrical signals here refer to electrical signals acquired by converting acoustic waves generated from optical pulses irradiated based on 7 bits of the code string in the example above. In other words, it could be said that the decoding using such a predetermined number of electrical signals may be decoding using electrical signals corresponding to a predetermined number of decode strings. The predetermined number here refers to the number corresponding to the number of the code string, which is 7 in this example. Therefore, in this example, the number of decode strings to be used for decoding is also equal to 7 (predetermined number). In the example above, because the probe is continuously scanned (moved) about the subject, that is, the relative positions of the subject and the probe change every one pulse irradiation, the electrical signals used for decoding contains redundant electrical signals that correspond to 6 bits of the code string (a21, a31, ... an1). As described above, according to this embodiment, the decode string corresponding to a predetermined number of code strings used for light irradiation is cyclically used to decode every 1-bit digital signal.

The decoding block calculates a post-decoding signal delayed by τL from an input digital signal.

While the light irradiation and decoding of received signals are being performed as described above, the transfer mechanism 013 moves the relative position of the probe 003 about the subject 000. As a result, decoded signals at times shifted by τL could be acquired at a plurality of locations. While TR is defined by the depth of a subject (such as 20 µsec or more when a subject at 1500 m/sec is observed at a depth of 30 mm), τL may be arbitrarily shortened. For example, τL may be 10 µsec or shorter when a light source is used which could be driven at a repetition frequency of 100 kHz or higher. In other words, applying the present invention allows reception of signals at many locations and directions within one observation time period independent of the depth of observation of a subject. By reconstructing an image by using signals acquired at many locations and directions, characteristic information of the subject may be provided with the resulting image having fewer artifacts. The time could also be reduced for acquiring data at the same number of locations and directions.

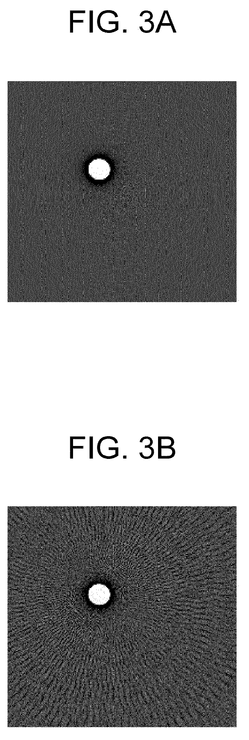

With reference to Figs. 3A and 3B, advantages of the present invention will be described. Figs. 3A and 3B illustrate reconstruction results acquired by using a model in which a point target is placed within an observation region and one receiving element (converting element) rotates 360 degrees around the point target to receive photoacoustic signals. Fig. 3A illustrates a reconstruction result according to the present invention. The time period τL between irradiated light pulses is 1 µsec, and the light irradiation is performed by using a predetermined number 511 of code strings. The predetermined number of code strings are repeatedly used 120 times while the receiving element (converting element) is rotating 360 degrees. In other words, a total of code strings of 61320 (511 x 120) bits are used, and an image is reconstructed by decoding every τL with the movement of the receiving element (converting element). Finally, signals received at 61320 directions are used for the reconstruction.

Fig. 3B illustrates a reconstruction result in a case where control is performed such that optical pulses for a predetermined number of 511 bits are irradiated where the time period τL between the pulses of the light irradiation is equal to that in the case illustrated in Fig. 3A. In this case the receiving element (converting element) is not moved until after the resulting acoustic waves are received, then the receiving element (converting element) is moved to the next position and optical pulses are then irradiated. The moving amount of the receiving element (converting element) is three degrees every time. A total number of used code strings is equal to that in the case in Fig. 3A, but the number of directions of reception is equal to 120 directions.

Comparing Figs. 3A and 3B, an artifact (a pattern spreading around a point target) of the reconstruction can be observed in Fig. 3B while it is found that such an artifact can be inhibited in the case according to the present invention as illustrated in Fig. 3A. An equal number of optical pulses are transmitted, and the imaging time period is also shorter according to the present invention (while a longer time period is necessary for the reception in the case in Fig. 3B), and it is found that an image with more reduced artifacts can be provided. Application of the present invention can provide a subject information acquiring apparatus which is capable of acquiring information at more directions and positions of reception in a shorter period of time, and reducing the number of artifacts.

While Fig. 1 illustrates the light irradiation position is defined separately from the probe and is not moved, the light irradiation position may be moved by the transfer mechanism 013 being a scanning unit in accordance with the movement of the position of the probe. In this case, an effect is provided that the signal-to-noise ratio of the region of interest can be improved.

Furthermore, in order to inhibit occurrence of a range sidelobe due to decoding within an observation region of a subject, the transmission time (which is a light irradiation time and corresponds to τL x (n-1) in this embodiment) using a predetermined number of code strings may be longer than the reception time (corresponding to TR in this embodiment) based on the observation depth. In other words, this means that the time period necessary for irradiating optical pulses coded by a predetermined number of pulse streams to a subject is longer than the time period necessary for receiving photoacoustic waves occurring from the subject in response to the irradiation of the coded optical pulses. This allows reduction of the number of artifacts.

An increased transmission time (light irradiation time) using a predetermined number of code strings may possibly be influenced by a deformation of the subject during the time. In this case, the transmission time (light irradiation time) using a predetermined number of code strings may be approximately equal to the reception time period (appropriately TR as described above) based on the observation depth. In other words, this means that the time period necessary for irradiating optical pulses coded by a predetermined number of pulse streams to a subject is substantially equal to the time period necessary for receiving by a converting element the photoacoustic waves occurring from the subject in response to the irradiation of coded optical pulses. This may reduce image influences of a change in shape of the subject such as a body motion caused by heartbeats, breathing and so on.

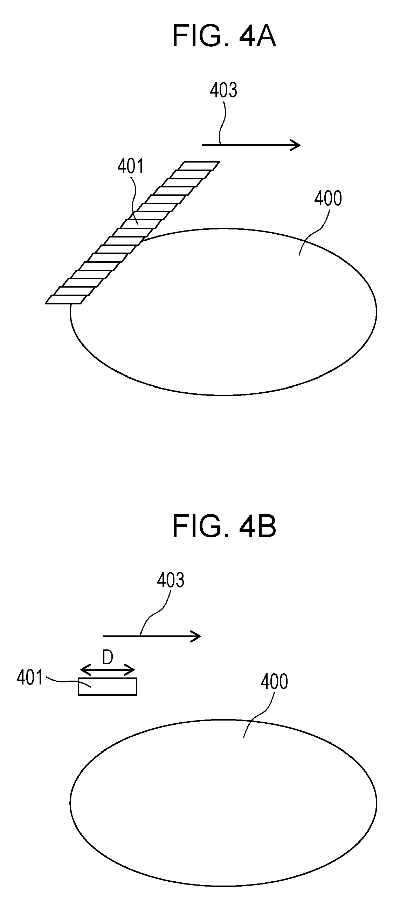

Figs. 4A and 4B illustrate another embodiment of the present invention.

Because the system overview is the same as that illustrated in Fig. 1, the description will be omitted, and differences therefrom will be described.

A light source radiates optical pulses to a subject 400 in accordance with code strings generated by the coding control block 002. A linear array probe 401 includes a plurality of receiving elements (converting elements), and the plurality of receiving elements (converting elements) are aligned in a one-dimensional direction and move in a direction 403 orthogonal to the direction of the alignment. According to this embodiment, the plurality of converting elements may receive photoacoustic waves concurrently from light irradiated once and may perform decoding processing thereon. The processing for reconstructing a photoacoustic image by using a plurality of decoded signals acquired by decoding acoustic waves received by the plurality of converting elements is the same as that in the aforementioned example.

Assume here that the velocity of movement of the linear array probe 401 in the direction 403 is V and the length of the aligned converting elements in the direction 403 is D. As described above, decoding for each 1 bit of coding allows generation of decoded signals received at a plurality of positions. For example, according to this embodiment, when decoding processing is performed by every 1 bit, a decoded signal received at every reception position V x τL may be acquired.

Signal reception at more positions and directions are preferable for reducing artifacts after the image is reconstructed. However, comparing a loose alignment of receiving elements (converting elements) (where a gap is provided between the receiving elements (converting elements)) and a tight alignment of the receiving elements (where the receiving elements (converting elements) are substantially in contact with each other), the tight alignment produces a higher effect of artifact reduction. In other words, the density of the reception positions for acquiring decoded signals may be equal to or higher than the density of the tight alignment.

In other words, when decoding processing is performed by every 1 bit, it may be controlled such that V x τL that is a position for acquiring a decoded signal could be substantially equal to or smaller than a pixel width D in the direction of movement, that is, (V x τL ≈ D) or (V x τL < D). In other words, a relationship of V x τL ≤ D is preferred. This means that the moving velocity V of a probe about a subject by means of a scanning unit, a time period τL between irradiations of optical pulses coded by a predetermined number of pulse streams, and a width D of a converting element in the direction of movement of the probe about the subject by means of the scanning unit, satisfies a relationship of V x τL <= D. In this case, the moving velocity V may be changed, or τL may be adjusted for control, or both of the parameters may be controlled. Under such control, an enhanced higher artifact reduction effect may be acquired.

Having described the decoding processing by every 1 bit above, the decoding processing may be performed on received signals by every 2 bits or digital signals delayed by 2 x τL. In this case, the scale of the signal processing may be reduced more than that of the decoding processing by every 1 bit. When decoding processing by every P bits is performed, the position for acquiring a decoded signal is V x τL x P. Thus, control may be performed so as to acquire (V x τL x P ≈ D) or (V x τL x P < D) a relationship between the direction of movement and the pixel width.

For continuous optical pulse irradiation, when the speed of storage of received data to a memory or the processing speed after that is not sufficient, the light irradiation or signal reception may be stopped before signals are completely acquired from the entire set region, and when storage to the memory and the subsequent processing complete, the light irradiation and signal reception may be resumed. It should be noted that the light irradiation and signal reception may be resumed after the position of the probe is returned to the position before the stop in order to keep coherence with the state before the stop. The expression, "position before the stop" may refer to a position to which the probe is returned by the amount corresponding to the sum of the time period necessary for transmitting a predetermined number of code strings, and the time period for receiving photoacoustic waves from a region where a photoacoustic signals is acquired, for example.

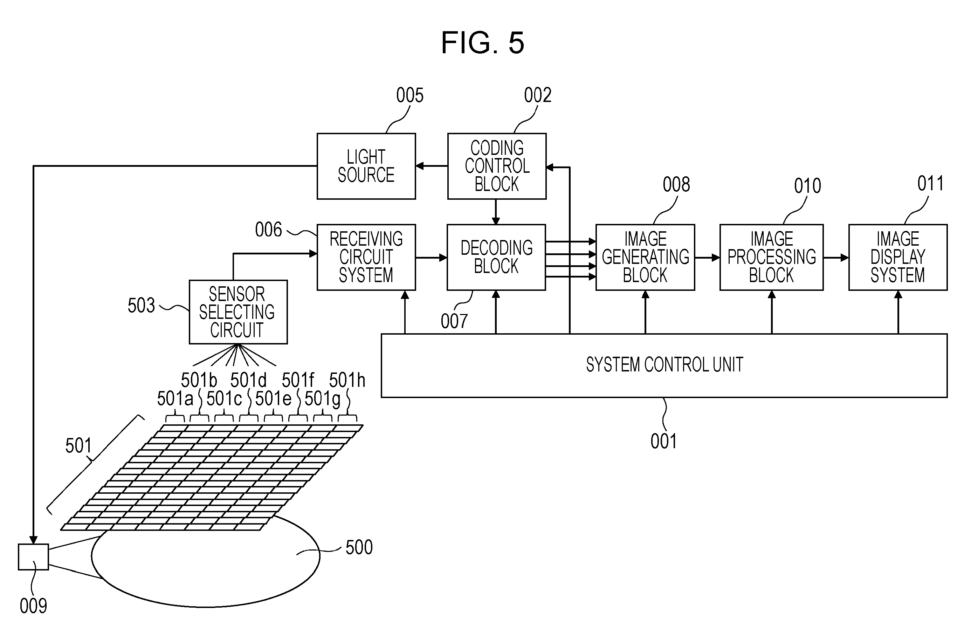

Fig. 5 illustrates another embodiment of the present invention.

Fig. 5 conceptually illustrates a system of the embodiment. Differences from those in Fig. 1 will only be described.

A light source radiates optical pulses to a subject 500 in accordance with code strings generated in the coding control block 002. A plurality of receiving elements (converting elements) receive photoacoustic waves by using two-dimensional probes 501 which are aligned two-dimensionally. The two-dimensional probe 501 is connected to a sensor selection circuit 503 which is a selecting unit. The sensor selection circuit transmits to the receiving circuit system 006 a result of synthesis of electrical signals (received signals) of a plurality of receiving elements in the two-dimensional probe 501 or a result of synthesis of signals of a plurality of receiving elements.

Operations of the sensor selection circuit 503 will be described below. The sensor selection circuit 503 selects a plurality of receiving elements in the two-dimensional probe 501. While light is being irradiated, a receiving element group included in the column 501a is selected, and a receiving element group included in a next column 501b is selected. Signals of the receiving element groups are transmitted to the receiving circuit system 006. These operations of the sensor selection circuit 503 are equivalent to relative movement of positions of receiving elements about the subject 500, providing the advantages of the present invention.

Performing this control may eliminate the necessity for concurrently receiving data of all receiving elements in a two-dimensional probe and allow inhibition of the processing scale of the system. According to the present invention, the time for receiving only may be omitted, and the optical pulse irradiation may be continuously performed. Therefore, the data acquisition from an imaging region may be completed in a short period of time.

According to another controlling method of this embodiment, while light is being irradiated, data of received signals of the column 501a and column 501b are added in the direction orthogonal to the column direction and are output, and data of received signals of the column 501b and column 501c are added next in the direction orthogonal to the column direction and are transmitted to the circuit system 006. Under this control, the continuity of received signals may be improved, compared with received data selected column by column. Having described the example in which data of two columns are added, data of two or more columns may be added also in the alignment. In this case, signals of the receiving elements are add by applying a weight thereto, and the continuity may be increased by changing the weight to be applied. Such control with a weight allows signal reception at a middle position of a receiving element to be equivalent to signal reception at the very position of the receiving element, and thus allows generation of signals received at more reception positions, and further provides the effect of artifact inhibition.

Having described above the probe having receiving elements (converting elements) aligned two-dimensionally, the same effect as described above may be acquired with a probe having loosely aligned receiving elements (converting element) or a probe having receiving elements (converting elements) aligned on a hemispherical plane. Combining the electronic changes of receiving elements and movement of the whole probe having receiving elements (converting elements) may also provide the effects of the present invention.

According to the present invention, a subject information acquiring apparatus is provided which allows reduction of artifacts.

While the present invention has been described with reference to exemplary embodiments, it is to be understood that the invention is not limited to the disclosed exemplary embodiments. The scope of the following claims is to be accorded the broadest interpretation so as to encompass all such modifications and equivalent structures and functions.

## Claims

1. A subject information acquiring apparatus comprising:
a light source (005) configured to radiate optical pulses coded by a predetermined number of pulse streams to a subject (000);
a probe (003) including at least one converting element configured to receive photoacoustic waves occurring from the subject (000) having been irradiated with the coded optical pulses, and configured to convert the received photoacoustic waves to electrical signals; and
information acquiring means for decoding a predetermined number of electrical signals of the electrical signals output from the converting element, and acquire characteristic information on the subject (000) based on the predetermined number of decoded electrical signals;
wherein the relative positions of the converting element included in the probe (003) and the subject (000) are changed before the radiation of the predetermined number of optical pulse streams completes; and
the predetermined number of electrical signals to be decoded by the information acquiring means are partially redundant before and after the change of the relative positions of the converting element and the subject (000).

2. The subject information acquiring apparatus according to claim 1, further comprising scanning means (013) for moving the probe (003) about the subject (000), wherein the scanning means changes the relative positions of the converting element included in the probe (003) and the subject (000).

3. The subject information acquiring apparatus according to claim 2, wherein the scanning means (013) moves an irradiation position for irradiating the optical pulses to the subject (000) about the subject (000).

4. The subject information acquiring apparatus according to claim 1, wherein the probe (003) includes a plurality of converting elements, the subject information acquiring apparatus further comprising selecting means for selecting partial converting elements from the plurality of converting elements, wherein the converting elements selected by the selecting means are changed to change the relative positions of the converting elements included in the probe (003) and the subject (000).

5. The subject information acquiring apparatus according to claim 1, wherein a time period necessary for irradiating optical pulses coded by the predetermined number of pulse streams to the subject (000) is longer than a time period necessary for receiving photoacoustic waves occurring from the subject (000) irradiated with the coded optical pulses by the converting element.

6. The subject information acquiring apparatus according to claim 1, wherein a time period necessary for irradiating optical pulses coded by the predetermined number of pulse streams to the subject (000) is substantially equal to a time period necessary for receiving photoacoustic waves occurring from the subject (000) irradiated with the coded optical pulses by the converting element.

7. The subject information acquiring apparatus according to claim 2, wherein a moving velocity V at which the scanning means moves the probe (003) about the subject (000), a time period τL between irradiations of optical pulses coded by the predetermined number of pulse streams, and a width D of the converting element in the direction of movement of the probe (003) about the subject (000), satisfy a relationship of V x τL ≤ D.

8. The subject information acquiring apparatus according to claim 1, wherein the probe (003) has a plurality of the converting elements.

9. The subject information acquiring apparatus according to claim 8, wherein the plurality of converting elements are aligned in one direction.

10. The subject information acquiring apparatus according to claim 8, wherein the plurality of converting elements are aligned in two directions.

11. The subject information acquiring apparatus according to claim 8, wherein the plurality of converting elements are aligned on a hemispherical plane.
